# FASCICULE DE BREVET EUROPEEN

(11) **EP 2 102 216 B1**
(45) Date de publication et mention de la délivrance du brevet: **21.07.2010**
(21) Numéro de dépôt: 07871822.8
(22) Date de dépôt: 10.12.2007
(51) Int. Cl.: C07D 495/04

(54) **DERIVE FLUORE DE OUINOLEINE-2(1H)-ONE, SON PROCEDE DE PREPARATION ET SON UTILISATION COMME INTERMEDIAIRE DE SYNTHESE**
FLUORINIERTE DERIVATE VON CHINOLIN-2(1H)-ON, VERFAHREN ZU IHRER HERSTELLUNG UND IHRE VERWENDUNG ALS SYNTHESE-ZWISCHENPRODUKT
FLUORINATED DERIVATIVE OF QUINOLEINE-2(1H)-ONE, METHOD FOR PREPARING THE SAME AND USE THEREOF AS A SYNTHESIS INTERMEDIATE

(30) Priorité: 12.12.2006 FR 0610802
(43) Date de publication de la demande: 23.09.2009
(73) Titulaire: Sanofi-Aventis, 75013 Paris (FR)
(72) Inventeur: LEGROUX, Didier, F-75013 Paris (FR); MORAGUES, Véronique, F-75013 Paris (FR); RUIZ-MONTES, José, F-75013 Paris (FR); SALLE, Laurent, F-75013 Paris (FR)
(74) Mandataire: Le Coupanec, Pascale A.M.P.
(86) Numéro de dépôt international: PCT/FR2007/002027
(87) Numéro de publication internationale: WO 2008/087277

(56) Documents cités:
- EP-A1- 0 574 313
- EP-A1- 1 078 928
- EP-B1- 0 850 235
- FR-A1- 2 738 823

## Description

La présente invention se rapporte à un nouveau dérivé fluoré de quinoléine-2(1*H*)-one, à son procédé de préparation, ainsi qu'à son utilisation comme intermédiaire de synthèse, en particulier pour la synthèse du 7-fluoro-2-oxo-4-[2-[4-(thiéno[3,2c]pyridin-4-yl)pipérazin-1-yl]éthyl]-1,2-dihydroquinoléine-1-acétamide.

Le 7-fluoro-2-oxo-4-[2-[4-(thiéno[3,2-*c*]pyridin-4-yl)pipérazin-1-yl]éthyl]-1,2-dihydroquinoléine-1-acétamide, ci-après dénommé « composé (II) », est décrit dans le brevet EP 0850235.

Ce composé est connu pour son intérêt en thérapeutique et notamment comme antagoniste de la sérotonine, plus particulièrement des récepteurs 4HT_{1B} et 5HT_{2A} , il peut être utilisé dans le traitement et la prévention de diverses formes de pathologies impliquant la sérotonine, comme les hypertensions artérielle, veineuse, pulmonaire, portale, rénale ou oculaire, les ischémies cardiaque, rénale, oculaire, cérébrale ou des membres inférieurs, l'insuffisance cardiaque, l'infarctus du myocarde, l'angor, les vasospasmes coronaires ou périphériques, les thromboses (seuls ou adjuvants à la thrombolyse), les artérites, la claudication intermittente, les resténoses après angioplastie et différents états pathologiques associés à l'athérosclérose, aux troubles de la microcirculation ou aux dysfonctionnements pulmonaires. Il peut être également utilisé, seul ou en association avec, d'autres substances dans les interventions de greffe vasculaire.

Le procédé de préparation du composé (II), tel que décrit dans le brevet EP 0850235, est le suivant:
1) réaction de la 4-(acétyloxy)-2*H*,3*H*-pyrane-2,6-dione avec la 3-fluoroaniline, à température ambiante et dans l'acide acétique pur, pour obtenir après lavage et séchage l'acide 3-(acétyloxy)-5-[(3-fluorophényl)amino]-5-oxopent-2-énoique,
2) cyclisation du composé obtenu précédemment, en présence d'un acide minéral tel que l'acide sulfurique concentré et pour obtenir l'acide 4-acétique-7-fluoroquinoléine-2(1*H*)-one (composé (III) ci-après),
3) estérification de l'acide ainsi obtenu pour obtenir le 4-acétate de méthyle-7-fluoro-quinoléine-2(1*H*)-one,
4) réduction de l'ester obtenu précédemment pour obtenir le 7-fluoro-4-(2-hydroxyéthyl)quinoléine-2(1*H*)-one,
5) activation de cet alcool par réaction avec le chlorure de thionyle pour obtenir le dérivé chloré, la 4-(2-chloroéthyl)-7-fluoro-quinoléine-2(1*H*)-one,
6) réaction du composé chloré ainsi obtenu avec la 4-(pipérazin-1-yl)thiéno[3,2-c]pyridine (composé (IV) ci-après) pour obtenir le 7-fluoro-4-[2-[4-(thiéno[3,2-c]pyridin-4-yl)pipérazin-1-yl]éthyl]quinoléine-2(1*H*)-one,
7) réaction du composé ainsi obtenu avec le bromoacétamide dans le tétrahydrofurane en présence d'hydroxyde de potassium et de bromure de tétrabutylammonium, aboutissant au 7-fluoro-2-oxo-4-[2-[4-(thiéno[3,2-c]pyridin-4-yl)pipérazin-1-yl]éthyl]-1,2-dihydroquinoléine-1-acétamide (composé (II) ci-après).

Les inventeurs se sont donnés pour but de parvenir à un nouveau procédé de synthèse de composé (II) utilisant un nouvel intermédiaire de synthèse.

L'invention a donc pour objet le composé de formule (I): 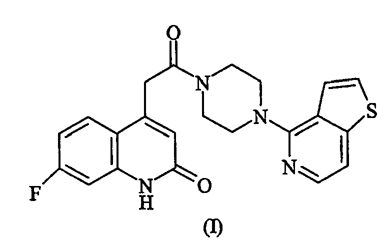 et ses sels pharmaceutiquement acceptables.

En effet, le composé de formule (I) peut exister à l'état de base ou salifié par des acides ou des bases, notamment des acides ou des bases pharmaceutiquement acceptables. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I), font également partie de l'invention.

Le composé de formule (I) peut également exister sous forme d'hydrates ou de solvats, à savoir sous forme d'associations ou de combinaisons avec une ou plusieurs molécules d'eau ou avec un solvant. De tels hydrates et solvats font également partie de l'invention.

Ce composé est particulièrement utile dans la synthèse de dérivés de quinoléin-2(1*H*)-one, notamment pour la synthèse du composé (II), tel que décrit dans le brevet EP 0850235, présentant la formule développée suivante :

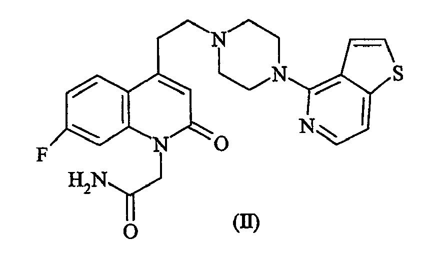

Selon le procédé en sept étapes décrit dans EP 0850235, on recueille le composé (II) avec un rendement chimique de 16% maximum à partir des produits de départ, à savoir la 4-(acétyloxy)-2*H*,3*H*-pyrane-2,6-dione et de la 3-fluoroaniline. Par ailleurs la réaction de chloration du 7-fluoro-4-(2-hydroxyéthyl)quinoléine-2(1*H*)-one (étape 5) décrite ci-avant est peu sélective et conduit à la formation, jusqu'à 25% en quantité molaire, d'un composé secondaire dichloré représenté ci-dessous :

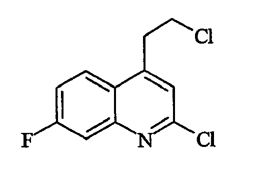

Par conséquent la mise au point d'un procédé pour la préparation du composé (II), comportant un nombre réduit d'étapes et fournissant un rendement suffisant en composé désiré reste d'un intérêt incontestable.

Or, on a découvert qu'il était possible d'utiliser un nouvel intermédiaire réactionnel, le 7-fluoro-4-[2-[4-(thiéno[3,2-*c*]pyridin-4-yl)pipérazin-1-yl]-2-oxoéthyl]-quinoléine-2(1*H*)-one, dénommé composé (I), pour synthétiser le composé (II). Le nouveau procédé utilisant l'intermédiaire (I) permet de synthétiser le composé (II) en un nombre réduit d'étapes par rapport au procédé antérieur connu, et avec un rendement nettement amélioré.

Selon l'invention, le composé (I) peut être synthétisé selon la figure 1. 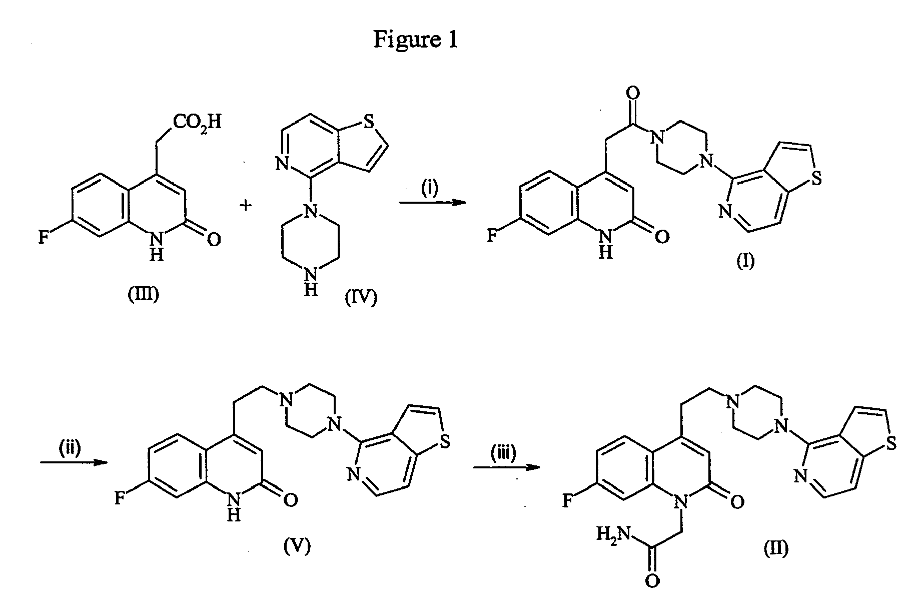 Le composé (I) peut être obtenu, dans une étape (i), par la réaction dans un solvant polaire aprotique tel que le tétrahydrofurane (THF), le méthyl-tétrahydrofurane, le toluène, l'acétonitrile, la N-méthylpyrrolidone (NMP) ou le diméthylformamide (DMF), de l'acide 4-acétique-7-fluoroquinoléine-2(1*H*)-one (III) avec la 4-(pipérazin-1-yl)thiéno[3,2-*c*]pyridine (IV) en présence d'un agent de couplage, présent en excès tel que le chlorure de pivaloyle, le chlorure de thionyle, le chloroformiate d'éthyle, le chloroformiate d'isobutyle de préférence le carbonyldiimidazole.

Les composés de départ (III) et (IV) sont disponibles dans le commerce ou décrits dans la littérature, notamment dans la demande EP 0850235, ou peuvent être préparés selon des méthodes qui y sont décrites ou qui sont connues de l'Homme du métier.

Le composé (I) est ensuite, dans une étape (ii), réduit par un hydrure métallique alcalin dans un solvant polaire ou apolaire comme, par exemple, l'hydrure de lithium et d'aluminium dans le tétrahydrofurane, le méthyl-tétrahydrofurane, le toluène, le borohydrure de zinc (ZnBH₄) dans le tétrahydrofurane, le borohydrure de sodium en présence d'un acide de Lewis tel que TiCl₄ ou AlCl₃ ou le borane selon des conditions connues de l'Homme de l'art, préférentiellement l'hydrure de lithium et d'aluminium dans le tétrahydrofurane; on obtient le 7-fluoro-4-[2-[4-[thiéno[3,2-*c*]pyridin-4-yl)pipérazin-1-yl]éthyl]quinoléine-2(1*H*)-one (V) précédemment évoqué lors de la synthèse du composé (II) décrite dans EP 0850235.

On réalise ensuite, dans une étape (iii), l'alkylation de l'amine secondaire à l'aide d'un agent éléctrophile ou alkylant par exemple de formule Halogène-CH₂-CO-NH₂ tel que des halogénures d'alkyle du type 2-bromoacétamide, 2-chloroacétamide, de préférence le 2-chloroacétamide, en présence d'une base telle que le carbonate de potassium, dans un solvant polaire aprotique tel que le tétrahydrofurane ou la potasse ou la soude, dans le diméthylformamide, en présence ou non d'un catalyseur de transfert de phase, tel que le bromure de tétrabutylammonium. On obtient le composé de synthèse (II).

Ainsi, selon le nouveau procédé conforme à l'invention on obtient le composé (V) à partir de l'acide 4-acétique-7-fluoro-quinoléine-2(1*H*)-one (III) et de la 4-(pipérazin-1-yl)thiéno[3,2-*c*]pyridine (IV) en deux étapes, avec des rendements de 99,3% et 64,6% respectivement pour chaque étape et avec un rendement pour ces deux étapes de 64%. Comparativement au brevet EP 0850235 le composé (V) est obtenu en quatre étapes à partir du composé (III), avec un rendement global de 30,5%.

Les exemples qui suivent illustrent l'invention sans la limiter. Les micro-analyses et les spectres IR, RMN et de masse confirment la structure des composés obtenus.

### EXEMPLE 1 : synthèse du composé (I)

### 1. Synthèse des produits de départ

### 1.1. 4-(pipérazin-1-yl)thiéno[3,2-c]pyridine (IV)

Dans un réacteur, on introduit 1 kg de 4-chlorothiéno[3,2-*c*]pyridine (1 éq.), 1,5 kg de pipérazine (3 éq.) et 8 L de 1-pentanol ou d'alcool isoamylique. On chauffe au reflux pendant 5 h, on refroidit et on filtre le chlorhydrate de pipérazine formé. On ajoute au filtrat 2,4 L d'eau et on acidifie avec de l'acide chlorhydrique concentré. Après décantation, la phase aqueuse est amenée à pH basique avec une solution de 2,3 L de soude 30 % et 2,6 L d'eau, puis extraite avec deux fois 1,3 L de toluène. On concentre la phase toluènique de moitié et on ajoute 3,5 L de méthylcyclohexane. On refroidit à 0°C, on filtre et on sèche le produit obtenu sous vide à 50°C. On obtient une poudre jaune pâle de composé (IV).
Rendement : 74 %
Analyses : HPLC = 99,2 %

### 1.2. Acide 4-acétique-7-fluoroquinoléine-2(1H)-one (III)

Comme décrit dans le brevet EP 0850235, à une solution agitée de 40,77 kg (366,9 moles) de 3-fluoroaniline dans 125 L d'acide acétique pur, on ajoute par petites portions, 62,32 kg (366,3 moles) de 4-(acétyloxy)-2*H*,3*H*-pyrane-2,6-dione. On agite le milieu réactionnel pendant deux heures à 30°C, on laisse refroidir à température ambiante et on dilue dans 375 L d'eau déminéralisée. On récupère le solide obtenu qui est essoré, lavé abondamment à l'eau et séché à l'étuve sous vide (40°C) pendant 48 heures.

On obtient 67,38 kg d'acide 3-(acétyloxy)-5-[(3-fluorophényl)amino]-5-oxopent-2-énoique sous forme d'une poudre.
Rendement : 65,4 %
Analyses : Point de fusion = 117°C

On verse par petites portions 67,38 kg (239,6 moles) d'acide 3-(acétyloxy)5-[(3-fluorophényl)amino]-5-oxopent-2-énoique dans 186 L d'acide sulfurique concentré à 15°C sous forte agitation, puis on chauffe le milieu réactionnel à 100°C pendant 90 minutes. Après refroidissement, on verse lentement cette solution sur 371 L d'eau déminéralisée à 15°C. On filtre le solide ainsi obtenu. On le lave à l'eau. On met le produit humide en suspension dans 154 L de DMF et on agite vigoureusement pendant une heure à 30°C. On filtre et on lave avec du DMF. On obtient le composé (III).
Rendement : 82,3 %
Analyses : HPLC = 97,3 %

### 2. 7-fluoro-4-[2-[4-(thiéno[3,2-c]pyridin-4-yl)pipérazin-1-yl]-2-oxoéthyl]-quinoléine-2(1H)-one (I)

Dans un réacteur, on introduit 56,95 kg (1 éq.) d'acide 4-acétique-7-fluoroquinoléine-2(1*H*)-one (III), puis 850 L de THF. On ajoute alors sous agitation, 49,2 kg (1,17 éq.) de carbonyldiimidazole. On chauffe à 45°C pendant 17 h, puis on additionne une solution de 56,9 kg (1 éq.) de 4-(pipérazin-1-yl)thiéno[3,2-*c*]pyridine (IV) dans 114 L de THF. On agite à 60°C pendant douze heures. On refroidit à 20°C, on filtre et on lave avec du THF. Le produit (I) n'est pas séché et il est directement engagé dans l'étape suivante.
Rendement : 99,3 %
Analyses : HPLC = 95,2 %
RMN 1H (DMSO, 300MHz) : δ 3,50 (m, 4H); 3,75(m, 4H); 4,05 (s, 2H); 6,37 (s, 1H); 7,05 (m, 2H); 7,5 (m, 2H); 7,70 (m, 1H); 7,79 (d, 1H); 8,04 (d, 1H); 11,77 (s, 1H).

### EXEMPLE 2 : synthèse du 7-fluoro-2-oxo-4-[2-[4-(thiéno[3,2-c]pyridin-4-ypipérazin-1-yl]éthtl]-1,2-dihydroquinoléine-1-acétamide (II)

### 1. 7-fluoro-4-[2-[4-[thiéno[3,2-c]pyridin-4-yl)pipérazin-1-yl]éthyl]-quinoléine-2(1H)-one (V)

Dans un réacteur, à une suspension de 22,2 kg (2,3 éq.) d'hydrure de lithium aluminium dans 580 L de tétrahydrofurane, on introduit en trente minutes à 10°C 107,7 kg (1 éq.) d'intermédiaire 7-fluoro-4-[2-[4-(thiéno[3,2-*c*]pyridin-4-yl)pipérazin-1-yl]-2-oxoéthyl]-quinoléine-2(1H)-one (I) puis 323 L de THF. On agite pendant trois heures à température ambiante, puis après contrôle de la fin de réaction, on refroidit et on ajoute successivement sans dépasser 15°C, 21,5 L d'eau, 21,5 L de soude 4N et 180 L d'eau. On agite une heure et on filtre les sels minéraux. On concentre à trois volumes de THF et on coule sur 1400 L d'eau. On filtre et on réempate dans 1000 L d'un mélange eau/THF : 3/7. On sèche sous vide à 50°C. On obtient le composé (V).
Rendement : 64,6 %
Analyses : HPLC = 98,5 %

### 2. 7-fluoro-2-oxo-4-[2-[4-(thiéno[3,2-c]pyridin-4-yl)pipérazin-1-yl] éthyl] 1,2-dihydroquinoléine-1-acétamide (II)

Dans un réacteur, on introduit 66,9 kg de 7-fluoro-4-[2-[4-[thiéno[3,2-c]pyridin-4-yl)pipérazin-1-yl]éthyl]-quinoléine-2(1H)-one (V), 18,4 kg de 2-chloroacétamide (1,2 éq.), 51,5 kg de carbonate de potassium (2 éq.) et 335 L de DMF. On agite à 50°C pendant vingt heures puis on refroidit à 25°C et on ajoute 1000 L d'eau. On filtre la suspension obtenue, on lave à l'eau et on réempate dans 307 L d'éthanol. On filtre le solide. On obtient 60,7 kg estimé sec d'un solide blanc (II).

## Revendications

1. 7-fluoro-4-[2-[4-(thiéno[3,2-c]pyridin-4-yl)pipérazin- 1 -yl]-2-oxoéthyl]-quino léine-2(1*H*)-one de formule (I): 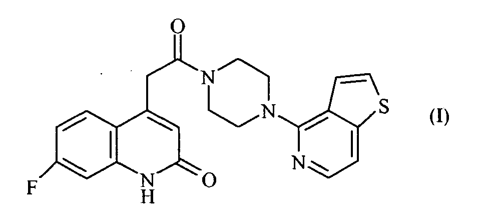 et ses sels pharmaceutiquement acceptables.

2. Composé de formule (I) selon la revendication 1, **caractérisé en ce qu'**il se présente à l'état d'hydrate ou de solvat.

3. Procédé de préparation du 7-fluoro-4-[2-[4-(thiéno[3,2-c]pyridin-4-yl)pipérazin-1-yl]-2-oxoéthyl]-quinoléine-2(1*H*)-one **caractérisé en ce que** l'on fait réagir l'acide 4-acétique-7-fluoroquinoléine-2(1*H*)-one (III) avec la 4-pipérazin-1-yl)thiéno[3,2-*c*]pyridine (IV).

4. Utilisation du 7-fluoro-4-[2-[4-(thiéno[3,2-c]pyridin-4-yl)pipérazin-1-yl]-2-oxoéthyl]-quinoléine-2(1*H*)-one de formule (I) comme intermédiaire dans la synthèse du 7-fluoro-2-oxo-4-[2-[4-[thiéno[3,2-c]pyridin-4-yl)pipérazin-1-yl]éthyl]-1,2-dihydroquinoléine-1-acétamide (II).

5. Procédé de préparation du 7-fluoro-2-oxo-4-[2-[4-[thiéno[3,2-*c*]pyridin-4-yl)pipérazin-1-yl]éthyl]-1,2-dihydroquinoléine-1-acétamide (II) **caractérisé en ce qu'**il comporte les étapes suivantes :
i) Réaction de l'acide 4-acétique-7-fluoroquinoléine-2(1*H*)-one (III) et de la 4-pipérazin-1-yl)thiéno[3,2-*c*]pyridine (IV),
ii) Réduction du 7-fluoro-4-[2-[4-(thiéno[3,2-*c*]pyridin-4-yl)pipérazin-1-yl]-2-oxoéthyl]-quinoléine-2(1*H*)-one (I),
iii) Alkylation de l'amine secondaire du composé réduit (V) grâce à un agent alkylant de type halogénure d'alkyle.

## Claims

1. 7-Fluoro-4-[2-[4-(thieno[3,2-c]pyridin-4-yl)piperazin-1-yl]-2-oxoethyl]quinolin-2(1*H*)-one of formula (I): 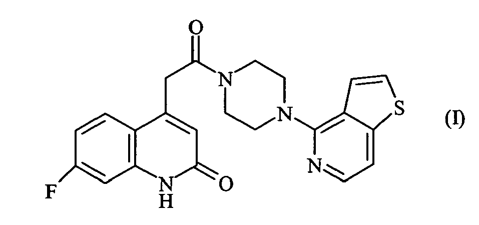 and the pharmaceutically acceptable salts thereof.

2. Compound of formula (I) according to Claim 1, **characterized in that** it is in the form of a hydrate or of a solvate.

3. Process for preparing 7-fluoro-4-[2-[4-(thieno[3,2-c]pyridin-4-yl)piperazin-1-yl]-2-oxoethyl]quinolin-2(1*H*)-one, **characterized in that** 4-(acetic acid)-7-fluoroquinolin-2(1*H*)-one (III) is reacted with 4-(piperazin-1-yl)thieno[3,2-*c*]pyridine (IV).

4. Use of 7-fluoro-4-[2-[4-(thieno[3,2-c]pyridin-4-yl)piperazin-1-yl]-2-oxoethyl]-quinolin-2(1*H*)-one of formula (I) as an intermediate in the synthesis of 7-fluoro-2-oxo-4-[2-[4-[thieno[3,2-c]pyridin-4-yl]piperazin-1-yl]ethyl]-1,2-dihydroquinoline-1-acetamide (II).

5. Process for preparing 7-fluoro-2-oxo-4-[2-[4-[thieno[3,2-*c*]pyridin-4-yl]piperazin-1-yl]ethyl]-1,2-dihydroquinoline-1-acetamide (II), **characterized in that** it comprises the following stages:
i) reaction of 4-(acetic acid)-7-fluoroquinolin-2(1*H*)-one (III) and 4-(piperazin-1-yl)thieno[3,2-*c*]pyridine (IV),
ii) reduction of 7-fluoro-4-[2-[4-(thieno[3,2-*c*]pyridin-4-yl)piperazin-1-yl]-2-oxoethyl]quinolin-2(1*H*)-one (I),
iii) alkylation of the secondary amine of the reduced compound (V) by means of an alkylating agent of alkyl halide type.

## Patentansprüche

1. 7-Fluor-4-[2-[4-(thieno[3,2-c]pyridin-4-yl)-piperazin-1-yl]-2-oxoethyl]chinolin-2(1*H*)-on der Formel (I): 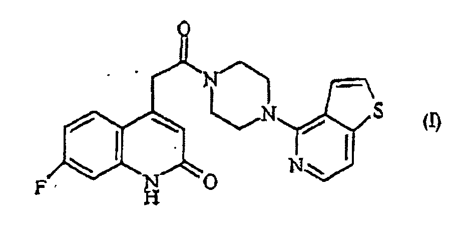 und pharmazeutisch unbedenkliche Salze davon.

2. Verbindung der Formel (I) nach Anspruch 1, **dadurch gekennzeichnet, daß** sie in Form eines Hydrats oder Solvats vorliegt.

3. Verfahren zur Herstellung von 7-Fluor-4-[2-[4-(thieno[3,2-c]pyridin-4-yl)piperazin-1-yl]-2-oxo-ethyl]chinolin-2(1*H*)-on, **dadurch gekennzeichnet, daß** man 4-Essigsäure-7-fluorchinolin-2(1*H*)-on (III) mit 4-(Piperazin-1-yl)thieno[3,2-*c*]pyridin (IV) umsetzt.

4. Verwendung von 7-Fluor-4-[2-[4-(thieno[3,2-c]-pyridin-4-yl)piperazin-1-yl]-2-oxoethyl]chinolin-2(1*H*)-on der Formel (I) als Zwischenprodukt bei der Synthese von 7-Fluor-2-oxo-4-[2-[4-[thieno-[3,2-c]pyridin-4-yl]piperazin-1-yl]ethyl]-1,2-dihydrochinolin-1-acetamid (II).

5. Verfahren zur Herstellung von 7-Fluor-2-oxo-4-[2-[4-[thieno[3,2-c]pyridin-4-yl]piperazin-1-yl]-ethyl]-1,2-dihydrochinolin-1-acetamid (II), **dadurch gekennzeichnet, daß** man:
i) 4-Essigsäure-7-fluorchinolin-2(1*H*)-on (III) und 4-(Piperazin-1-yl)thieno[3,2-c]pyridin (IV) umsetzt,
ii) 7-Fluor-4-[2-[4-(thieno[3,2-c]pyridin-4-yl)-piperazin-1-yl]-2-oxoethyl]chinolin-2(1*H*)-on (I) reduziert,
iii) das sekundäre Amin der reduzierten Verbindung (V) mit einem Alkylierungsmittel vom Alkylhalogenid-Typ alkyliert.
